# EUROPEAN PATENT APPLICATION

(11) **EP 3 320 909 A1**
(43) Date of publication of application: **16.05.2018**
(21) Application number: 15897610.0
(22) Date of filing: 08.07.2015
(51) Int. Cl.: A61K 36/889, A61K 36/87, A61K 36/23, A61K 38/18, A61K 38/08, A61K 31/522, A61P 17/14

(54) **PHARMACEUTICAL COMPOSITION THAT PROMOTES CAPILLARY REGENERATION**

(71) Applicant: Armesso Am España, S.L., 08003 Barcelona (ES)
(72) Inventor: ARIZA ANDRADE, Eduardo, 08003 Barcelona (ES); CHACÓN LÓPEZ, Carlos Eduardo, 08003 Barcelona (ES)
(74) Representative: Urizar Anasagasti, Jesus Maria
(86) International application number: PCT/ES2015/070530
(87) International publication number: WO 2017/005942

(57) **Abstract**

A pharmaceutical composition that promotes hair regeneration, which is provided in a liquid and stable state, and is useful for treating alopecia, stimulating hair regeneration and providing improved hair growth with lower incidence of hypochromic anaemia, the active ingredient of which is formed by a fivefold combination of concentrations of allopathic active ingredients, hydroglycolic or hydroalcoholic vegetable extracts, homeopathic dilutions of trace elements, growth factors of biotechnological origin and biomimetic peptides, transported in a vehicle that is pharmaceutically appropriate for intradermal administration or topical application, in a direct manner or by means of devices for cellular stimulation via radiation or micro-galvanic current. Optionally, the pharmaceutical composition can be combined in situ with autologous-origin growth factors.

## Description

### Object of the invention

The present invention discloses a liquid and stable pharmaceutical composition, useful for treating alopecia, stimulating hair regeneration and providing improved hair growth with lower incidence of hypochromic anaemia, whose active ingredient consists of a fivefold combination of concentrations of allopathic active ingredients, hydroglycolic or hydroalcoholic vegetable extracts, homeopathic dilutions of trace elements, growth factors of biotechnological origin and biomimetic peptides, transported in a pharmaceutically appropriate vehicle for intradermal administration or topical application, directly or by means of devices for cellular stimulation via radiation or micro-galvanic current. Optionally, the pharmaceutical composition can be combined in situ with autologous-origin growth factors.

### Background of the invention

Several documents in the state of the art disclose combined formulations for the treatment of alopecia. For example, US 6,596,266 B2 (Natural Science, Inc.) discloses formulations based on Minoxidil as the active ingredient in combination with potentiating elements for hair regeneration in cases of male baldness. This document, besides being limited to certain cases of androgenic alopecia, does not use growth factors or trace elements, nor claims to improve the quality of regenerated hair or facilitate regeneration thereof with lower incidence of hypochromic anaemia.

US 7,704,533 B2 (J&J Consumer Companies) discloses compositions and methods for inducing hair growth using *Coggygria* extracts by means of topical application. These formulations, in addition to lacking growth factors and allopathic components, have localised effects, limited to the area of topical application, are not suitable, for pharmaceutical reasons, for intradermal administration, and do not even mention the need to provide improved strength and lower incidence of hypochromic anaemia.

CO 29.341 (AMD HAIR, S.A.) discloses a stable formulation for treating alopecia and promoting hair regeneration, based on a stable double combination of active ingredients of allopathic origin, in association with a second component of homeopathic origin, consisting of a decimal dilution of trace elements. This document, however, did not address a growing need in the capillary pharmaceuticals market: provide a regeneration process which, besides being sustained and enduring, improves the strength, tensile strength and discoloration of the regenerated hair fibre. The formulation disclosed in said patent was limited to the use of two sets of components and, while it provided quantitative improvements in regeneration, did not represent a qualitative technical leap in these aspects.

WO2013/167927 (Biotix) discloses a composition for treating alopecia based on biotin, minerals and trace elements, organic silicon, pentoxyphylline and procaine hydrochloride. The formulation disclosed, besides only being suitable for medical administration at specialist clinics, and therefore lacking industrial application, does not make reference to the ranges of its constituent components nor demonstrates advances with regard to strengthening the regenerated hair or delaying its discoloration process.

US 2012/0003300 (Pangaea Laboratories, Ltd.), while including biomimetic peptides combined with an antifungal molecule, is intended to combat capillary infections caused by fungi and bacteria. Similarly, US 9006291 (Pharma Patent Holding, Inc.) discloses a hair growth molecule incorporated in one or more pharmaceutical preparations for hair growth included in a kit exclusively for topical use.

And lastly, WO2012065065 (Follica, Inc.) claims a comprehensive treatment method for regulating hair growth and scalp scar revision using one or more substances alone or following a defined therapeutic sequence.

Said documents differ from the present invention in that the invention, in addition to involving active ingredients of varying nature, is suitable for treating alopecia and promoting hair regeneration, not for combating dandruff or coadjuvating in the healing of wounds. Likewise, none of these documents disclose injectable formulations for intradermal or mesotherapeutic use.

### Description of the invention

In view of the limitations of earlier compositions, the inventors addressed the challenge of providing a formulation suitable for sustainably generating new, stronger and more resistant hair, with lower incidence of hypochromic anaemia. To this end, specific tests were designed, aimed at determining the viability of using a fivefold combination of active ingredients which, besides including a basic combination of allopathic and homeopathic components, incorporates three new groups of active ingredients suitable for solving the problems of known formulations: 1) selected hydroglycolic or hydroalcoholic vegetable extracts; 2) growth factors of biotechnological origin and, optionally, exclusively for in situ recomposition purposes, of autologous origin; and 3) biomimetic peptides synthesised in molecular sequencers.

Thus, firstly, experiments including Finasteride in a conventional dual combination were performed. This molecule was discarded upon determining its potential for generating unwanted side effects or medical interactions, including those related to decreased libido, erectile dysfunction and development of breast tissue, consistent with findings by Sato and Takeda in their paper published in 2011 "Evaluation of efficacy and safety of finasteride 1 mg in 3,177 Japanese men with androgenetic alopecia."

Next, laboratory tests were conducted adding active promoter drugs and vascular activators, such as aspirin, clopidrogel and ticlopidine, and antioxidant drugs and molecules, such as ascorbic acid and lycopene. These tests, in all cases, resulted in chemical and physicochemical instability in the form of unwanted oxidation and precipitation reactions that rendered it unviable from a medical and commercial perspective. The most relevant instabilities included, namely, the colouring of transparent solutions, which took on dark amber tones, caused by unexpected oxidation or chemical changes in the ascorbic acid, with immediate and subsequent precipitation when the tests were subjected to accelerated stability studies.

Thus, by discarding the addition of the active agents in question, the inventors initiated the experimental incorporation of Growth Factors ("GF"), understood as protein-type molecules which act directly on specific cell receptors and allow them to activate defence and growth mechanisms which enable them to achieve physiological balance, called cell homeostasis.

Firstly, so-called autologous-origin growth factors were considered and discarded -for industrial formulation purposes, but not as an optional active ingredient for their optional addition in situ- since, due to being obtained from patient plasma, they were only suitable for application in a single patient, in individual and custom treatments, usually performed in doctor's offices. Therefore, although the addition of these GFs was discarded in the formulation reproducible on industrial scale, their consideration and experimentation as an optional component for use in reconstitution in doctor's offices and specialist hair treatment centres was maintained upon obtaining evidence of their potentiating effect on the claimed formulation.

Thus, the research was focused on experimentation with growth factors synthesised by biotechnological production from human DNA replicas copied by cloning and subsequently applied to bacteria that secrete proteins by fermentation, which are then isolated and purified. Of the large number of growth factors of this kind, known per se in the state of the art, arising from the manipulation of eukaryotic cells maintained in culture, the following may be cited in accordance with the classification made by Gospodarowicz & Moran in 1976 and by Jiménez de Asua in 1980:
a) Platelet-derived growth factor: "PDGF";
b) Transforming growth factor-beta: "TGF-beta";
c) Fibroblast growth factor: "FGF";
d) Epidermal growth factor: "EGF";
e) Hepatocyte growth factor: "HGF";
f) Vascular epithelial growth factor: "VEGF";
g) Type I insulin-like growth factor: "IGF-I";
h) Granulocyte colony-stimulating growth factor: "G-CSF".

Such growth factors share specific characteristics for performing their stimulating functions, namely that relating to fusion with receptor molecules located on the plasma membrane of the target cells, generating signals which are subsequently transmitted towards the interior of the cytoplasm through the activation of enzymes with kinase activity, which generate a phosphorilation reaction of certain protein substrates. This reaction generates a cascade of signals, known as transduction, which activates the gene expression and produces the specific transcription of certain genes that will be translated in the ribosomes, becoming the cellular response to a certain growth factor (Carpenter & Cohen, 1979).

Regarding the epidermal growth factor (EGF), it was discovered in 1962 by Stanley Cohen at the University of Vanderbilt, a discovery that earned him the Nobel Prize in Medicine and Physiology in 1986 (Hall, 2009). A patent for the use of EGF as a cosmetic was granted to Greg Brown in 1989 (Brown, 1997). The human EGF corresponds to a molecular weight of 6025 Da. Additionally, it has three disulphide bonds in its intramolecular configuration (Carpenter & Cohen, 1990) and is encoded by a gene located in chromosome 4 (Massagué & Pandiella, 1993; Boonstra et al., 1995). This factor plays an important role in the regulation of cell growth, proliferation, differentiation and survival (Herbst, 2004). It was also discovered that EGF is a salivary iodine regulating agent and may also act maintaining the integrity of gastric tissue, among other functions (Venturi & Venturi, 2009).

Both EGF and TGF-alpha have been found to have the same receptor known as EGFR, which in humans is encoded by the c-erbB1 gene on the short arm of chromosome 7 (Tsui & Farral, 1991) and which consists of a family of integral glycoproteins from the plasma membrane which have 1,186 amino acids with a molecular weight of 170 KDa consisting of an amino-terminal extracellular domain, a trans-membrane domain and a carboxyl-terminal cytoplasmic domain where the catalytic site responsible for the intrinsic kinase tyrosine activity (Hernández-Sotomayor & Carpenter, 1992) called HER1, Erb B1 or simply EGRF (Kumar et al., 2005), is located.

EGF has also been shown to have high affinity to its receptor on the cell surface, instantly stimulating a tyrosine-kinase activity that initiates a transduction signal, generating an amplification of this signal by way of cascade resulting in a multitude of cytosolic modifications such as a significant increase in calcium levels, increase in the glycolytic and protein synthesis mechanism and increase in the expression of certain genes, including the gene for the synthesis of both EGFR itself and also for the synthesis of DNA and, therefore, for cell proliferation, differentiation and survival, prevention or induction of apoptosis and cell motility (Fallon et al., 1984). (1)

After a careful assessment of the aforementioned properties, the inventors determined that the Growth Factors suitable for inclusion in the formulation should be those which have demonstrated their cellular activity in cell regeneration processes in the hair follicle, being:
1) *bFGF* or basic fibroblast growth factor, which is the stimulator of the angiogenesis or vascular creation by chemotactic action on endothelial cells;
2) *IGF* or insulin-like growth factor, also known as somatomedin C, which is a protein whose chemical structure is similar to insulin, and plays a key role in children's growth. In humans its production is stimulated by the growth hormone (GH) and, in turn, is a more potent natural activator of PKB signal transduction, a cell growth and proliferation stimulator. In addition to being a potent inhibitor of programmed cell death, it has been proven to be an important factor in the regulation of cell growth and development and cellular DNA synthesis; and
3) *VEGF* or endothelial vascular growth, whose main function in humans is to control the formation of new blood vessels, the protection and proliferation of endothelial cells, and the hyperpermeability of blood vessels.

After defining the selection of growth factors of biotechnological origin suitable for inclusion in the formulation and the possibility of optionally adding, in situ, a certain dose of autologous-origin GF thereto, laboratory experimentation was initiated with substances of known hair strengthening and regeneration activity including, inter alia, some metabolic process-promoting peptides and nucleotides, organic and inorganic substances with physicochemical characteristics suitable for generating synergy or increased effectiveness in hair regeneration. Experiments were also conducted with vegetable extracts of known activity on the scalp and hair follicle including, inter alia, *Aloe vera,* rosemary, chamomile and *Centella asiatica,* subsequent to which vegetable extracts with antioxidant properties were used including, inter alia, green tea and *Vitis vinifera* or grape extract.

The inventors then addressed the challenge of evaluating the complex chemical nature of the aforementioned vegetable extracts, starting with those known per se for their beneficial effects on hair, such as *Aloe vera,* which provides nutrients, vitamins, minerals and substances with capillary activity such as triterpenes, tannins and saponins. Tests were also conducted with specific extracts of an antioxidant and vascularising nature, whose beneficial properties for the sustainability of hair colour in adults and/or youths with premature greying were especially attractive for the desired purposes, with emphasis on the evaluation of *Vitis vinifera* or grape extract and *Centella asiatica.*

Firstly, it was considered that the presence of tannins and terpenoids in *Centella asiatica* transmits this substance of recognised cardiovascular effects. Although its use as a tonic and application as a coadjuvant in the treatment of tired legs, varicose veins and haemorrhoids has been document in literature, there are no references to its use alone or combined as an antioxidant, nor to its potentiating effect for sustaining colour in hair with premature greying.

Moreover, the grape (*Vitis vinifera*), of ancient tradition in human consumption, is known for its unique content in chemical substances of high antioxidant power (vitamins, hypoalexins, flavonoids, polyunsaturated fatty acids) that makes it effective against cellular senescence. However, there are no bibliographic references to its use alone or combined in hair cosmetics nor relative to its capacity to prevent or delay hair depigmentation.

Therefore, after conducting clinical tests that will be explained below, the inventors decided to incorporate a homogeneous combination or blend containing standardised hydroglycolic vegetable extracts of *Vitis vinifera* and *Centella asiatica* to the formulation, the therapeutic purpose of which in the formulation is to prevent or delay the depigmentation of regenerated and existing hair. The effect achieved by adding the selected vegetable extracts is surprising, since their presence in the formulation delays the onset of premature greying or allows hair to regenerate with its melamine structure, with less presence or absence of grey hair, which opens new avenues to prevent and attack gradual greying due to ageing or premature greying of hair. The antioxidant properties and mixtures of amino acids and peptides present in these extracts, combined with the high degree of chemical compatibility between them, make it possible to achieve the purpose of preventing peroxide build-up on the follicles and slowing unwanted hair discoloration.

Lastly, it was determined that, in order to supplement the activity of the GFs of biotechnological origin, it was viable to add two peptides of synthetic origin, of known biological regeneration activity, to the composition as potentiators and stimulators of extracellular matrix synthesis and catalysts of biological reactions that inhibit or promote cellular communication and metabolic processes. To this end, a mixture of biomimetic peptides was selected from acetyl hexapeptide and 3-octapeptide and L-methionine, made synthetically by directed molecular sequencing, which would be present as part of the active ingredient of the formulation.

It was then defined that the active ingredient of the formulation would be formed from a fivefold combination which, in addition to comprising basic allopathic and homeopathic components, would include selected growth factors, hydroglycolic or hydroalcoholic vegetable extracts and biomimetic synthetic peptides, transported in a pharmaceutically acceptable isotonic liquid carrier, suitable for intradermal or topical administration, even by means of devices for cellular stimulation via radiation or micro-galvanic current. Subsequently, the possibility of combining autologous growth factors in situ for application in custom hair treatments was determined.

It was then determined that the formulations comprise, as the active ingredient, a set of components with therapeutic activity summarised as follows:
1) Active ingredients of an allopathic nature, in defined concentrations and selected from or corresponding to Biotin, Pentoxyphylline, D-Panthenol and Minoxidil.
2) Active trace elements presented in decimal solutions of a homeopathic nature, prepared from mother tinctures from inorganic salts containing silicon, gold, calcium, cobalt, copper, iron, iodine, chrome, lithium, magnesium, manganese, chlorine, sulphur and phosphorus.
3) Human Growth Factors obtained and purified biotechnologically, including synthetic peptides synthetically purified by directed sequencing comprising an acetyl hexapeptide and an octapeptide.
4) Natural standardised extracts of vegetable species including Saw *palmetto, Vitis vinifera* and *Centella asiatica.* The use of autologous-origin growth factors has been defined as a fifth optional ingredient for incorporation in situ.
5) A mixture of biomimetic peptides selected from acetyl hexapeptide and 3-octapeptide and L-methionine prepared synthetically by directed molecular sequencing.

After defining the active ingredients, the inventors addressed the challenge of providing an isotonic, sterile and stable carrier suitable for transporting the formulation and simultaneously allowing the topical and intradermal administration thereof, based on two relevant variables: physicochemical and microbiological stability of the components, given by the obtainment of a transparent liquid, without presence of perceptible suspended particles, and the prolonged stability of certain measurable characteristics including, inter alia, content, degradation levels, pH, density and microbiological quality.

It was thus decided to conduct tests using a pharmaceutically suitable isotonic carrier, composed of diluents, pH stabilisers and sequestrants, in addition to using methods for manufacturing sterile liquid pharmaceutical preparations based on cutting-edge technologies to guarantee asepsis and the consequent suitability of intradermal parental administration, without generating adverse side effects. As a result of their experiments on this front, the inventors obtained satisfactory natural stability data over a period of 28 months for the product that is the object of the patent application, in accordance with the tests specified below.

The pharmaceutical carrier suitable for bringing the formulation to specific irrigation points (inactive follicles or in progressive phase of inactivation) intradermally was achieved by means of the systematic selection of the excipients or formulation aids approved by the USP (United States Pharmacopeia) for parenteral liquid forms, resulting in a stabilised solution with near-isotonicity gradients comprising sodium chloride, disodium EDTA, dibasic sodium phosphate, ethyl alcohol, propylene glycol and water-for-injection-quality hot water (USP WFI).

Similarly, successful results were obtained in the preclinical studies and tests conducted to determine the safety, cytotoxicity and toxicity of the formulation, including satisfactory results in terms of irritability and hypoallergenicity. Said studies and tests were commissioned to and executed with accredited institutions, in strict compliance with international standards for this type of tests, with the aim of corroborating the effectiveness and activity of the formulation that is the object of the application.

With regard to the allopathic component of the formulation, it should be noted that Minoxidil is used as an optional component for its known and previously mentioned therapeutic action on agents such as Finasteride, which had drawbacks, adverse effects and contraindications. Similarly, it should be noted that Pentoxyphylline helps improve blood supply to the affected area and was selected from among various drugs for its chemical compatibility with other agents such as theophylline and phentolamine.

As regards the standardised hydroglycolic or hydroalcoholic vegetable extracts present in the formulation, *Saw palmetto* extract was selected for its activity comparable to (hormonal-type) inhibitors of 5-alpha reductase and its non-hormonal nature. Biotin and D-Panthenol were selected for their known benefits for the hair fibre in its various growth phases. Hydroglycolic extracts of *Centella asiatica* and grape were selected for their compatibility and vascularising therapeutic and antioxidant activity.

Furthermore, the 14 components that constitute the homeopathic component of the formulation generate, due to their association in the proposed decimal percentages, a joint benefit as coadjuvants in the treatment of impaired cellular functions. The simultaneous association of such components has been designed creating a therapeutic scale in dilutions ranging from medium-low (D6) to medium-high (D30). Said dilutions are mainly associated with the purpose of functioning in a single set as a coadjuvant in the treatment of the cellular functions impaired by trace element deficiency, which regulate cellular metabolism and are evident in states of weakness and fatigue, hair loss and skin disorders.

The intradermal or topical administration of the claimed compositions allows the active inhibitors of 5-alpha reductase to remain in contact with the areas around the hair follicle, which are key to start the process of regeneration and regression of the inactivation thereof, aided by specific Growth Factors such as, in this case, a known mixture of concentration of fibroblastic growth factor, insulin-like growth factor and vascular endothelial growth factor, in combination with a specific hair nutrition system (vitamins) and a synergistic mixture (homeopathic composition of a homeostatic nature) produce surprising results in terms of effectiveness against premature hair loss in men and women never before observed or obtained by the compounds currently in the market.

### Formulation Example No. 1

| INGREDIENT | CONCENTRATION % w/w |
|---|---|
| SODIUM CHLORIDE | 0.520 |
| DISODIUM EDTA | 0.015 |
| DIBASIC SODIUM PHOSPHATE | 0.035 |
| ETHYL ALCOHOL | 0.150 |
| PROPYLENE GLYCOL | 0.150 |
| BIOTIN | 0.016 |
| PENTOXYPHYLLINE | 0.027 |
| D-PANTHENOL | 0.150 |
| PURPLE GRAPE EXTRACT | 0.350 |
| CENTELLA ASIATICA EXTRACT | 0.750 |
| SAW PALMETTO EXTRACT | 0.350 |
| ACIDUM SILICICUM (D8) | 0.095 |
| AURUM METALLICUM (D10) | 0.095 |
| CALCIUM CARBONICUM (D8) | 0.095 |
| COBALTUM NITRICUM (D30) | 0.095 |
| CUPRUM METALLICUM (D12) | 0.095 |
| FERRUM METALLICUM (D10) | 0.095 |
| IODUM (D10) | 0.095 |
| KALIUM BICHROMICUM (D6) | 0.095 |
| LITHIUM CARBONICUM (D10) | 0.095 |
| MAGNESIUM CHLORATUM (D6) | 0.095 |
| MANGANUM SULPHURICUM (D10) | 0.095 |
| NATRIUM CHLORATUM (D8) | 0.095 |
| PHOSPHORUS (D8) | 0.095 |
| SULPHUR IODATUM (D8) | 0.095 |
| MIXTURE OF F.C BFGF, IGF AND VEGF | 0.0000002 |
| ACETYL HEXAPEPTIDE | 0.0000001 |
| 3-OCTAPEPTIDE | 0.0000001 |
| L-METHIONINE | 0.0000001 |
| WATER FOR INJECTION W.F.I. C.S. | 100 |

### Formulation Example No. 2

| INGREDIENT | CONCENTRATION % w/w |
|---|---|
| SODIUM CHLORIDE | 0.520 |
| DISODIUM EDTA | 0.015 |
| DIBASIC SODIUM PHOSPHATE | 0.035 |
| ETHYL ALCOHOL | 0.150 |
| PROPYLENE GLYCOL | 0.150 |
| BIOTIN | 0.016 |
| PENTOXYPHYLLINE | 0.027 |
| D-PANTHENOL | 0.150 |
| MINOXIDIL | 0.085 |
| PURPLE GRAPE EXTRACT | 0.350 |
| CENTELLA ASIATICA EXTRACT | 0.750 |
| SAW PALMETTO EXTRACT | 0.350 |
| ACIDUM SILICICUM (D8) | 0.095 |
| AURUM METALLICUM (D10) | 0.095 |
| CALCIUM CARBONICUM (D8) | 0.095 |
| COBALTUM NITRICUM (D30) | 0.095 |
| CUPRUM METALLICUM (D12) | 0.095 |
| FERRUM METALLICUM (D10) | 0.095 |
| IODUM (D10) | 0.095 |
| KALIUM BICHROMICUM (D6) | 0.095 |
| LITHIUM CARBONICUM (D10) | 0.095 |
| MAGNESIUM CHLORATUM (D6) | 0.095 |
| MANGANUM SULPHURICUM (D10) | 0.095 |
| NATRIUM CHLORATUM (D8) | 0.095 |
| PHOSPHORUS (D8) | 0.095 |
| SULPHUR IODATUM (D8) | 0.095 |
| MIXTURE OF F.C BFGF, IGF AND VEGF | 0.0000002 |
| ACETYL HEXAPEPTIDE | 0.0000001 |
| 3-OCTAPEPTIDE | 0.0000001 |
| L-METHIONINE | 0.0000001 |
| WATER FOR INJECTION W.F.I. C.S. | 100 |

### Formulation Example No. 3

| INGREDIENT | CONCENTRATION % w/w |
|---|---|
| SODIUM CHLORIDE | 0.520 |
| DISODIUM EDTA | 0.015 |
| DIBASIC SODIUM PHOSPHATE | 0.035 |
| ETHYL ALCOHOL | 0.150 |
| PROPYLENE GLYCOL | 0.150 |
| BIOTIN | 0.016 |
| PENTOXYPHYLLINE | 0.027 |
| D-PANTHENOL | 0.150 |
| MINOXIDIL | 0.085 |
| PURPLE GRAPE EXTRACT | 0.350 |
| CENTELLA ASIATICA EXTRACT | 0.750 |
| SAW PALMETTO EXTRACT | 0.350 |
| ACIDUM SILICICUM (D8) | 0.095 |
| AURUM METALLICUM (D10) | 0.095 |
| CALCIUM CARBONICUM (D8) | 0.095 |
| COBALTUM NITRICUM (D30) | 0.095 |
| CUPRUM METALLICUM (D12) | 0.095 |
| FERRUM METALLICUM (D10) | 0.095 |
| IODUM (D10) | 0.095 |
| KALIUM BICHROMICUM (D6) | 0.095 |
| LITHIUM CARBONICUM (D10) | 0.095 |
| MAGNESIUM CHLORATUM (D6) | 0.095 |
| MANGANUM SULPHURICUM (D10) | 0.095 |
| NATRIUM CHLORATUM (D8) | 0.095 |
| PHOSPHORUS (D8) | 0.095 |
| SULPHUR IODATUM (D8) | 0.095 |
| MIXTURE OF F.C BFGF, IGF AND VEGF | 0.0000002 |
| ACETYL HEXAPEPTIDE | 0.0000001 |
| 3-OCTAPEPTIDE | 0.0000001 |
| L-METHIONINE | 0.0000001 |
| AUTOLOGOUS PLATELET-RICH PLASMA EXT. | 0.75 |
| WATER FOR INJECTION W.F.I. C.S. | 100 |

### Preparation of the product on industrial scale

1-PREPARATION OF THE BASE SOLUTION: In a reactor of appropriate volume under agitation at a speed no higher than 600 RPM, at room temperature, add the necessary amount of cold WFI (water for injection), adding, in the indicated order, the pharmaceutical isotonicity excipients. Check for complete dissolution and commencement of filtered nitrogen bubbling.
2-ADDITION OF THE ALLOPATHIC COMPONENT: Without suspending agitation and nitrogen bubbling, in specific order and one by one, add all the active ingredients of the allopathic component until dissolution; for some substances a solubilisation system that involves mixing pharmaceutical-grade solvents that guarantee the solubility and homogeneity of the system must be incorporated.
3-ADDITION OF THE HOMEOPATHIC COMPONENT: Under constant agitation and nitrogen bubbling, add the homeopathic dilutions that constitute the formulation, previously treated and diluted with the Hahnemannian method.
4-ADDITION OF THE VEGETABLE EXTRACTS: Without suspending agitation and nitrogen bubbling, incorporate the vegetable extracts one by one as indicated, check solubility and discard the solution in the event of physicochemical phenomena such as precipitation or change in colour of the product.
5-ADJUSTMENT OF pH: Measure pH and adjust if necessary using hydrochloric acid or DMAE (dimethylaminoethanol) solutions, as appropriate.
6-AJUSTMENT OF VOLUME OF PREPARATION: Top up to volume with the preparation, stir for 3 minutes and check the homogeneity of the mixture. Measure the final pH of the solution.
7-STERILISING FILTRATION: Use membranes or cartridges in a 0.45 and 0.22 micron sterilising filtration system, filtering previously using 20 micron prefilters.
8-ADDITION OF THE BIOMIMETIC PEPTIDES AND GROWTH FACTORS: Under aseptic conditions and following all the applicable GMPs for managing bioclean areas, add the peptides to the final volume of the preparation filtered by cartridges and/or sterilisers.
9-FILLING: Perform automatic filling and sealing under aseptic conditions following BPM (GMP).
10-SAMPLING AND COMPLETE ANALYSES: Perform sampling of the products and conduct all the tests required to release the product (analysing sterility, pyrogen, content, pH, appearance, etc.).
11-OPTICAL CONTROL AND BAGGING: Perform optical control of the entire product to guarantee a clean product free from foreign particles.
12-RELEASE AND TAKING OF RETENTION SAMPLES: Once the analysis results are satisfactory, proceed to release the product and store the corresponding retention samples of the manufactured batch together with the Quality Assurance Department.

When the product that is the object of the invention is administered intradermally, it is preferably applied at a rate of 1 mL per syringe, injecting several times, wherein each injection delivers approximately 0.05 to 0.1 ml, and throughout the affected area (scalp). The frequency of the treatment varies depending on different factors and taking into account the shape and complexity of the alopecia or degree of hair loss, but in general it has been determined that the obtainment of positive results depends on the administration of at least 10 applications over 12 weeks of continuous treatment, with treatment intervals defined by observation and medical criteria. In the presence of the optional in situ addition of autologous GFs, it was determined that the regenerative effect, after delivering the same treatment sessions and intervals, was increased significantly.

The application of the disclosed formulations has demonstrated an effectiveness percentage in men and women, measured on the basis of the arrest of hair loss and consequent hair regeneration, more than 60%, when in the available clinical literature the effectiveness of pharmaceutical compositions for treating alopecia-related pathologies, either comprising a single agent or comprising a combination of agents (never in allopathic/homeopathic mixtures such as those claimed), does not exceed 30% of effectiveness. Furthermore, combined with the fact that the use of the intended formulations, due to the accurate determination of the ranges of the allopathic and homeopathic active ingredients, has no constraints, even in women and children, as in the case of various formulations available in the market or revealed in literature, their inventive nature and contribution to the treatment of alopecic pathologies is undisputable.

## Claims

1. A pharmaceutical composition that promotes hair regeneration, based on a combination of allopathic agents comprising Pentoxyphylline, Biotin and D-Panthenol, and a combination of homeopathic dilutions, **characterised in that** it further comprises the following components:
a) A combination of standardised vegetable extracts composed of:
(i.) *Saw palmetto* extract;
(ii.) Purple grape extract; and
(iii.) *Centella asiatica* extract;
b) A combination of isolated and purified Growth Factors, consisting of bFGF, IGF and VEGF; and
c) A mixture of biomimetic peptides selected from acetyl hexapeptide and 3-octapeptide and L-methionine.

2. The pharmaceutical composition, according to claim 1, **characterised in that** the combination of vegetable extracts is comprised between 0.025 and 0.70%, the combination of Growth Factors is comprised between 0.0000002% and 0.0000009% w/w and the mixture of biomimetic peptides is present in a concentration range comprised between 10 and 50 PPM for each peptide.

3. The pharmaceutical composition, according to the preceding claims, **characterised in that** the active ingredient is present in a range comprised between 1.40% and 3.64% w/w.

4. The pharmaceutical composition, according to the preceding claims, **characterised in that** the pharmaceutically acceptable isotonic liquid carrier is present in a range comprised between 98.60% and 96.36% w/w.

5. The pharmaceutical composition, according to the preceding claims, **characterised in that** it comprises, as an optional allopathic component, Minoxidil in a concentration range comprised between 0.05% and 1.5% w/w, increasing the total active ingredients of the formula in a range comprised between 1.90% and 5.14% and adjusting the value of the pharmaceutical carrier in a range comprised between 98.06% and 94.86% w/w.

6. The pharmaceutical composition, according to the preceding claims, **characterised in that** it is transported in a pharmaceutically acceptable isotonic carrier composed of sodium chloride, disodium EDTA, dibasic sodium phosphate, ethyl alcohol, propylene glycol and water-for-injection-quality water.

7. A method for treating alopecia and promoting hair regeneration, **characterised in that** the pharmaceutical composition of the preceding claims is combined in situ, prior to the application thereof, with autologous-origin growth factors extracted from platelet-rich plasma of the patient to be treated, in adequate amounts according to the nature and speed of the treatment.
